# EUROPÄISCHE PATENTANMELDUNG

(11) **EP 1 249 500 A1**
(43) Veröffentlichungstag der Anmeldung: **16.10.2002**
(21) Anmeldenummer: 01109174.1
(22) Anmeldetag: 12.04.2001
(51) Int. Cl.: C12Q 1/68, G01N 33/543

(54) **Verfahren zum Nachweis einer Substanz**

(71) Anmelder: Chimera Biotec GmbH, 28209 Bremen (DE)
(72) Erfinder: Niemeyer, Christof M., 28213 Bremen (DE); Adler, Michael, 27607 Langen-Debstedt (DE)
(74) Vertreter: Eisenführ, Speiser & Partner

(57) **Zusammenfassung**

Die Erfindung betrifft ein Verfahren zum Nachweisen einer Substanz in wässriger Lösung. Dabei wird eine Oberfläche bereitgestellt, die mit einer ersten Nucleinsäure versehen ist. Weiter wird ein Fänger-Reagens bereitgestellt. Das Fänger-Reagens umfasst eine zweite Nucleinsäure, die mit der ersten Nucleinsäure hybridisieren kann, und ein erstes Bindungsmolekül, das an die nachzuweisende Substanz binden kann, wobei die zweite Nucleinsäure und das erste Bindungsmolekül miteinander verbunden sind. Daneben wird ein Nachweis-Reagens bereitgestellt. Das Nachweis-Reagens umfasst ein zweites Bindungsmolekül, das an die nachzuweisende Substanz binden kann, und eine dritte Nucleinsäure, die mit dem zweiten Bindungsmolekül verbunden ist. Die mit er ersten Nucleinsäure verbundene Oberfläche, das Fänger-Reagens, das Nachweis-Reagens und eine wässrige Lösung der nachzuweisenden Substanz werden zusammengebracht, um ein Nachweiskonjugat aus Fänger-Reagens, nachzuweisender Substanz und Nachweis-Reagens zu bilden und dieses durch Hybridisieren von erster und zweiter Nucleinsäure an die Oberfläche zu binden und/oder an der Oberfläche zu bilden, wo die dritte Nucleinsäure nachgewiesen wird.

## Beschreibung

Die Einführung von Antikörper- oder Rezeptor-basierten Immunoassays in den 60er und 70er Jahren führte zur Entwicklung einer Vielzahl hochspezifischer Verfahren, um niedrigkonzentrierte Analytsubstanzen empfindlich und präzise nachzuweisen. So beschreibt J.R. Crowther in ELISA: Theory and Practice, Humana Press Inc., 1995, dass mit Enzym-verstärkten Assays oder Radioimmuno-Assays Substanzen mit Konzentrationen bis in den Attomol-Bereich (1 amol=1 x 10⁻¹⁸ mol) nachgewiesen werden können. Die Entwicklung hochwirksamer Wirkstoffe und Behandlungsmethoden der modernen Medizin, aber auch das Auftreten neuer Krankheiten wie die über das Prionprotein PrP^{Sc} nachweisbaren Encephalopathien (beispielsweise die bovine spongiforme Encephalopathie, Scrapie, Creutzfeld-Jacob-Krankheit, Gerstmann-Straussler-Scheinker-Syndrom) erfordern jedoch die Entwicklung von Nachweisverfahren, um niedrigstkonzentrierte Substanzen, möglichst im Subattomol- oder Zeptomolbereich (1 zmol= 1 × 10⁻²¹ mol), nachzuweisen.

Ferner besteht ein generelles, bislang ungelöstes Problem darin, niedrig- und niedrigstkonzentrierte Analytsubstanzen aus komplexen Medien wie Blutplasma und -serum mit hoher Empfindlichkeit und Sicherheit nachzuweisen.

T. Sano et al., *Science* 1992, 120 bis 122, beschreiben ein Immuno-PCR genanntes Verfahren, bei dem eine Polymerase-Kettenreaktion (PCR) als Verstärker-Reaktion benutzt wird, um Proteine mit einer Nachweisgrenze von bis zu 6 x 10⁻²² mol zu erfassen. Dabei wurde Rinderserum-Albumin (BSA) als Antigen-Modellsubstanz in Mikrotiterplatten immobilisiert, mit einem für BSA spezifischen biotinylierten Antikörper markiert und in einem nachfolgenden Inkubationsschritt über eine rekombinante Proteinchimäre aus Protein A und Streptavidin mit einem biotinylierten Marker-DNA-Fragment verknüpft. Anschließend konnte eine Nachweisgrenze bis zu 6 x 10⁻²² mol des mit dem immobilisierten Antigen verknüpften DNA-Fragments durch PCR erreicht werden. Dieses Verfahren ist jedoch, wie C.M. Niemeyer in *Biospektrum* 1999, 486, beschrieben hat, sehr aufwendig und ohne erhebliche Modifikationen praktisch nicht reproduzierbar.

H. Zhou et al., *Nucleic Acids Res. 1993,* 6038 bis 6039, stellten für die Immuno-PCR den signalerzeugenden Komplex aus Antikörper und Marker-DNA durch sequentielle Inkubation aus den Einzelkomponenten her, indem an den antigenspezifischen (Primär-) Antikörper zunächst ein biotinylierter (Sekundär-) Antikörper, dann Streptavidin und erst daran anschließend die biotinylierte Marker-DNA gebunden wurde. Die Immuno-PCR konnte auf diese Weise mit kommerziell erhältlichen Reagenzien durchgeführt werden. Nachteilig ist jedoch, dass die Empfindlichkeit des Nachweisverfahrens aufgrund der vielen Kopplungsschritte wesentlich verschlechtert und seine praktische Durchführung sehr erschwert wird.

C.M. Niemeyer et al., *Nucleic Acids Res.* 1999, 4553 bis 4561, beschreiben ein Nachweisverfahren, bei dem die nachzuweisende Substanz mit einem zuvor gebildeten supramolekularen Nachweis-Reagens markiert wird, wobei das Nachweis-Reagens ein Komplex aus durch Streptavidin oligomerisierten biotinylierten DNA-Markerfragmenten und biotinylierten Antikörpern gegen die nachzuweisende Substanz ist. Die im Nachweis-Reagens mit der nachzuweisenden Substanz verknüpften DNA-Markerfragmente wurden durch PCR nachgewiesen. Im Vergleich zu einem herkömmlichen ELISA konnte die Nachweisgrenze um etwa den Faktor 1000 gesenkt werden. Das Verfahren ist jedoch noch immer nur schwer handhabbar und nicht empfindlich genug. Auch die darin beschriebenen Waschschritte sind wegen der damit verbundenen Einbußen in der Nachweisempfindlichkeit nachteilig.

Es war eine Aufgabe der Erfindung, ausgehend von dem vorgenannten Stand der Technik ein weiteres Verfahren zum Nachweisen niedrigstkonzentrierter Substanzen anzugeben. Mit dem Nachweisverfahren sollte vorzugsweise nicht nur das Vorliegen und/oder die Abwesenheit einer Substanz nachgewiesen werden können (qualitativer Nachweis), sondern auch die Menge und/oder Konzentration der nachzuweisenden Substanz bestimmbar sein (quantitativer Nachweis).

Es war eine weitere Aufgabe, Nachweisverfahren anzugeben, die mit möglichst wenigen Waschschritten durchgeführt werden können.

Eine weitere Aufgabe war es, Nachweisverfahren anzugeben, die für möglichst viele unterschiedliche nachzuweisende Substanzen, beispielsweise Proteine, Peptide, Zucker, Lipide und dergleichen einsetzbar sind.

Die genannten Aufgaben werden gelöst durch ein Verfahren zum Nachweis einer Substanz in wässriger Lösung, umfassend die Schritte:
a) Bereitstellen einer Oberfläche mit einer damit verbundenen ersten Nucleinsäure,
b) Bereitstellen eines Fänger-Reagens, umfassend eine zweite Nucleinsäure, die mit der ersten Nucleinsäure hybridisieren kann, und ein erstes Bindungsmolekül, das an die nachzuweisende Substanz binden kann, wobei die zweite Nucleinsäure und das erste Bindungsmolekül miteinander verbunden sind,
c) Bereitstellen eines Nachweis-Reagens, umfassend ein zweites Bindungsmolekül, das an die nachzuweisende Substanz binden kann, und eine dritte Nucleinsäure, die mit dem zweiten Bindungsmolekül verbunden ist,
d) Zusammenbringen der mit der ersten Nucleinsäure verbundenen Oberfläche, des Fänger-Reagens, des Nachweis-Reagens und einer wässrigen Lösung der nachzuweisenden Substanz, um ein Nachweiskonjugat aus Fänger-Reagens, nachzuweisender Substanz und Nachweis-Reagens zu bilden und dieses durch Hybridisieren von erster und zweiter Nucleinsäure an die Oberfläche zu binden und/oder an der Oberfläche zu bilden,
e) Nachweisen von dritter Nucleinsäure, soweit sie in einem an die Oberfläche gebundenen Nachweiskonjugat vorliegt.

Aus C.M. Niemeyer et.al., *Analytical Biochemistry* 1999, 54 bis 63, ist bereits ein DNA-getriebenes Immobilisierungsverfahren (DNA-directed immobilization, DDI) bekannt. Dabei wird an einer Oberfläche eine vorgewählte Menge einer ersten einzelsträngigen Nucleinsäure angebracht. Ein biotinyliertes Protein, insbesondere ein biotinyliertes Immunglobulin, wird über Streptavidin mit einem zweiten biotinylierten DNA-Einzelstrang zu einem Präkonjugat verknüpft, wobei der zweite DNA-Einzelstrang komplementär zum ersten Einzelstrang ist. Durch Hybridisierung von erstem und zweitem DNA-Einzelstrang wird das jeweils mit dem ersten DNA-Einzelstrang verknüpfte Protein an der Oberfläche immobilisiert.

Überraschenderweise hat sich herausgestellt, dass DDI-Fänger-Reagenzien (entsprechend dem Präkonjugat der letztgenannten Veröffentlichung) nicht nur das Binden von Nachweis-Reagenzien nicht behindern, sondern sogar zu einer Verbesserung, das heißt Erniedrigung, der Nachweisgrenze führen können.

Durch die Verwendung von DDI-Fänger-Reagenzien kann zudem die Immobilisierung der nachzuweisenden Substanz an eine Oberfläche vorteilhaft lange hinausgezögert werden. Zudem können die Fänger-Reagenzien in Lösung, also ohne die Einschränkungen, denen sie bei einer herkömmlichen vorangehenden Immobilisierung unterliegen, an der nachzuweisenden Substanz binden. Beides ermöglicht eine Verbesserung der Nachweisgrenze.

Eine Oberfläche im Sinne des erfindungsgemäßen Verfahrens kann jede mit einer Nucleinsäure (dazu siehe unten) verbindbare Oberfläche sein. Insbesondere kann es sich um eine Glas-, Silizium- oder Metalloberfläche oder eine andere, vorzugsweise zur Herstellung von Biochips geeignete Oberfläche handeln. Unter den Metalloberflächen sind Gold- oder goldbeschichtete Oberflächen bevorzugt. Es kann sich jedoch auch um Kunststoffoberflächen, insbesondere Polystyrol- oder Polycarbonat-Oberflächen handeln, wie sie insbesondere in Mikrotiterplatten-Kavitäten vorliegen. Bei der Oberfläche kann es sich auch um die Oberfläche vorzugsweise magnetischer Mikropartikel handeln, wie sie zur Extraktion von Nucleinsäuren aus Flüssigkeiten verwendet werden.

Die Oberfläche kann mit die Verfahrensdurchführung nicht störenden Substanzen beschichtet (geblockt) werden. Insbesondere kann die Oberfläche mit Gelatine, Milchpulver und/oder Rinderserum-Albumin (BSA) beschichtet sein. Die Oberfläche kann auch mit Nucleinsäuren versehen sein, soweit diese die Durchführung des Nachweisverfahrens nicht beeinträchtigen.

Bindungsmoleküle im Sinne des Nachweisverfahrens sind solche Substanzen oder Substanzgemische, die unter den gewählten Verfahrensbedingungen vorzugsweise selektiv an die nachzuweisende Substanz binden können. Bindungsmoleküle können insbesondere Rezeptoren wie Protein A und Enzyme sein, aber auch mono- und/oder polyclonale Immunglobuline, insbesondere solche vom Typ G, und deren Fragmente sein. Solche Fragmente sind insbesondere bekannt unter den Bezeichnungen Fab, F(ab)₂, dsFv-Fragmente, scFv-Fragmente und Single-Chain-Antikörper. Des weiteren können Bindungsmoleküle auch niedermolekulare Stoffe sein wie Peptide, Peptoide, Haptene und Nucleinsäure-Bindungsreagenzien wie Aptamere. Die Verwendung polyclonaler Immunglobuline als Bindungsmolekül des Fänger- und/oder Nachweis-Reagens ist wegen ihrer im Vergleich zu monoclonalen Immunglobulinen häufig höheren Stabilität und häufig leichteren Verfügbarkeit bevorzugt. Die Fänger- und/oder Nachweisreagenzien umfassen, wenn das Bindungsmolekül ein Substanzgemisch mehrerer Substanz-Varianten wie beispielsweise ein polyclonales Immunglobulin ist, jeweils in einem Molekül mehrere der Substanz-Varianten (beispielsweise mehrere der Varianten eines polyclonalen Immunglobulins) oder sind jeweils Gemische aus Fänger- bzw. Nachweis-Reagenzien, deren Moleküle sich voneinander jeweils in ihren Bindungsmolekül-Varianten (beispielsweise den einzelnen Varianten eines polyclonalen Immunglobulins) unterscheiden.

Nucleinsäuren im Sinne des Nachweisverfahrens sind Substanzen, die eine oder mehrere Nucleobasen (Adenin, Cytosin, Guanin, Thymin, Uracil) oder deren funktionale Analoga, wie zum Beispiel Hypoxanthin, umfassen. Die Nucleobasen sind dabei vorzugsweise mit einem Zucker, insbesondere mit einer Pentose, Pentopyranose oder Pentofuranose verbunden. Besonders bevorzugt sind dabei Ribose und Desoxyribose. Die Zucker wiederum sind vorzugsweise über Phosphodiester-Bindungen miteinander verknüpft. Der Begriff Nucleinsäure umfasst auch sogenannte Peptid-Nucleinsäuren, bei denen das Zuckerphosphat-Rückgrat durch Aminosäuren, die durch Peptidbindungen miteinander verbunden sind, ersetzt ist (PNAs, siehe dazu beispielsweise E. Uhlmann, *Biol. Chem.* 1998, 1045-1052; P. Neilsen, *Acc. Chem. Res.,* 1999 (32), 624-630), und Pyranosyl-Nucleinsäuren wie p-RNAs (sie dazu beispielsweise M. Bolli, R. Micura, A. Eschenmoser, *Chem. Biol.* 1997(4), 309-320).

Die erste und zweite Nucleinsäure können unter den Verfahrensbedingungen jeweils miteinander hybridisieren. Dabei ist es bevorzugt, wenn sie unter den Verfahrensbedingungen keine internen Schleifen bilden können, wenn es also keine zwei Abschnitte jeweils einer Nucleinsäure gibt, die miteinander stabil hybridisieren können. Ebenfalls ist es bevorzugt, wenn die erste und zweite Nucleinsäure unter den gewählten Verfahrensbedinungen keine stabilen Homodimere bilden, so dass also keine zwei Moleküle der ersten Nucleinsäure miteinander stabil hybridisieren und/oder keine zwei Moleküle der zweiten Nucleinsäure stabil miteinander hybridisieren.

Die Nucleinsäuren sind jeweils mit der Oberfläche bzw. dem ersten/zweiten Bindungsmolekül derart verbunden, dass sie sich von ihren jeweiligen Bindungspartnern unter den gewählten Verfahrensbedingungen im wesentlichen nicht lösen, das heißt, dass während der Durchführung des Verfahrens weniger als 25% der Bindungen zerfallen. Besonders bevorzugt ist es, wenn die Nucleinsäuren und das erste und/oder zweite Bindungsmolekül und gegebenenfalls die Oberfläche biotinyliert sind, so dass die Nucleinsäuren über Streptavidin mit ihrem jeweiligen Bindungspartner verbunden werden können. Freie Biotin-Bindungsstellen von Streptavidin sollten anschließend durch Zugabe von Biotin abgesättigt werden, um störende Nebenreaktionen zu vermeiden.

Die Länge der ersten und zweiten Nucleinsäure beträgt vorzugsweise 5 bis 50 Nucleotide, die Länge der dritten Nucleinsäure beträgt vorzugsweise 50 bis 500 Nucleotide oder Basenpaare. Soll die dritte Nucleinsäure durch ein rolling-circle-Verfahren (siehe dazu beispielsweise B. Schweitzer et al., *Proc. Natl. Acad. Sci.* 2000, 10113-10119) oder ein anderes Verfahren nachgewiesen werden, bei dem eine geringere Länge der Nucleinsäure erforderlich ist, so beträgt die Länge der dritten Nucleinsäure vorzugsweise 5 bis 50 Nucleotide oder Basenpaare.

Der qualitative und vorzugsweise quantitative Nachweis der dritten Nucleinsäure kann, gegebenenfalls unter Einschalten eines Waschschrittes, auf zahlreiche verschiedene Weisen erfolgen. Insbesondere kann die dritte Nucleinsäure radioaktiv markiert sein/werden und radiographisch nachgewiesen werden. Sie kann jedoch auch mit Nucleinsäure-bindenden Farbstoffen, insbesondere Fluoreszenz-Farbstoffen, nachgewiesen werden. Dabei ist es besonders bevorzugt, wenn die Farbstoffe über eine zu einem Abschnitt der dritten Nucleinsäure komplementäre Sonde an die dritte Nucleinsäure gebunden sind bzw. werden. Die Sonde kann auch, wie in herkömmlichen Verfahren üblich, mit Enzymen verbunden sein, die selbst leicht nachweisbar sind oder die die Erzeugung eines leicht nachweisbaren Produkts bewirken.

Mit dem erfindungsgemäßen Verfahren können überraschend niedrige Nachweisgrenzen erzielt werden.

Ferner erlaubt es das Verfahren, das an die Oberfläche gebundene Fänger-Reagens wieder abzulösen und somit die Oberfläche zu regenerieren und das Fänger-Reagens, gegebenenfalls mit daran gebundener nachzuweisender Substanz, erneut in Lösung zu bringen.

Das Verfahren erlaubt es ferner, preisgünstige Materialien zu seiner Durchführung zu verwenden. Insbesondere Nucleinsäuren, auch biotinylierte Nucleinsäuren, sind preiswert und schnell erhältlich.

Ferner können die Nucleinsäuren weitgehend unabhängig von den Bindungsmolekülen, der nachzuweisenden Substanz und der verwendeten Oberfläche ausgewählt werden. Dies erlaubt es, die im Verfahren verwendeten Nucleinsäuren aufeinander abgestimmt zu standardisieren.

Das Verfahren ermöglicht es außerdem, die Reihenfolge des Zusammenbringens von Fänger-Reagens, Nachweis-Reagens und nachzuweisender Substanz sowie der mit der ersten Nucleinsäure verbundenen Oberfläche weitgehend frei zu wählen und so an die nachzuweisende Substanz anzupassen, dass eine möglichst niedrige Nachweisgrenze erreicht wird.

Bevorzugt ist ein Verfahren, bei dem Schritt d) wie folgt durchgeführt wird:
f) Zusammenbringen des Fänger-Reagens, des Nachweis-Reagens und der nachzuweisenden Substanz zum Herstellen eines Nachweiskonjugats aus Fänger-Reagens, nachzuweisender Substanz und Nachweis-Reagens, und anschließend
g) Zusammenbringen der mit der ersten Nucleinsäure verbundenen Oberfläche mit dem Nachweiskonjugat, um das Nachweiskonjugat durch Hybridisieren von erster und zweiter Nucleinsäure an die Oberfläche zu binden.

Es hat sich herausgestellt, dass die Anlagerung eines Nachweis-Reagens an eine nachzuweisende Substanz behindert sein kann, wenn diese bereits in immobilisierter Form vorliegt. Nach dem Immobilisieren der nachzuweisenden Substanz wird zudem zweckmäßigerweise ein Waschschritt zum Entfernen nicht immobilisierter Stoffe durchgeführt, um die Spezifität des Nachweisverfahrens zu steigern. Beides führt zu einer Verschlechterung der Nachweisgrenze.

Es ist daher wünschenswert, ein Nachweisverfahren so zu gestalten, dass (a) die Immobilisierung der nachzuweisenden Substanz möglichst spät im Verfahren erfolgt oder (b) auf andere Weise bewirkt wird, dass die Immobilisierung der nachzuweisenden Substanz an eine Oberfläche die Bindung eines Nachweis-Reagens an die nachzuweisende Substanz wenig oder nicht behindert.

Beides ist mit der bevorzugten Verfahrensgestaltung auf vorteilhaft einfache Weise möglich.

Insbesondere ist es bevorzugt, eine Lösung der nachzuweisenden Substanz mit einer Mischung aus Fänger- und Nachweis-Reagens zu vereinigen, bevor das Fänger-Reagens über seine zweite Nucleinsäure mit der an die Oberfläche gebundenen ersten Nucleinsäure hybridisiert. Auf diese Weise wird erreicht, dass ein hoher Anteil von Fänger- und Nachweis-Reagens an die nachzuweisende Substanz binden kann, so dass die Nachweisgrenze vorteilhaft erniedrigt ist. Außerdem ist es auf diese Weise möglich, die nachzuweisende Substanz durch eine vorgefertigte Mischung von Fänger- und Nachweis-Reagens in ein Nachweiskonjugat zu überführen. Für häufig nachzuweisende Substanzen lassen sich so die Anzahl der zur Durchführung des Verfahrens benötigten Lösungen minimieren sowie die Lösungen selbst vereinheitlichen und vorfabrizieren.

Besonders bevorzugt ist ein Verfahren, bei dem zum Nachweisen gemäß Schritt e) die dritte Nucleinsäure amplifiziert wird.

Zur Amplifikation von Nucleinsäuren sind eine Reihe von Verfahren bekannt. Zu den bekannten Verfahren gehören insbesondere die Polymerase-Kettenreaktion (PCR), rolling-circle-Amplifikation (RCA), Ligase-Kettenreaktion (LCR) und nucleic-acid-sequence-based-Amplifikation (NASBA). Diese ermöglichen es, auch kleinste Mengen einer (dritten) Nucleinsäure bis hin zu wenigen hundert, zehn oder gar einem einzigen Molekül zu vervielfältigen. Dadurch kann das Vorliegen eines einzigen oder weniger Moleküle der dritten Nucleinsäure qualitativ und/oder quantitativ nachgewiesen werden.

Zur Vervielfältigung der dritten Nucleinsäure und gegebenenfalls zu deren Nachweis sind insbesondere PCR-Verfahren geeignet und bevorzugt. Die Durchführung solcher Verfahren ist seit langem gängige Praxis, so dass die zur Durchführung einzustellenden Verfahrensparameter anhand einfacher Vorversuche oder anhand des allgemeinen Fachwissens leicht optimiert werden können. Ferner sind die zur Durchführung solcher Verfahren benötigten Substanzen verhältnismäßig preiswert sowie leicht und schnell erhältlich. Die zur Durchführung der Amplifikation benötigten Geräte sind zudem in der überwiegenden Zahl der molekularbiologischen Forschungsinstitute und/oder Arbeitsgruppen bereits vorhanden.

Ferner ist es bevorzugt, wenn das Fänger-Reagens umfasst:
- zwei oder mehr mit einem ersten Bindungsmolekül verbundene zweite Nucleinsäuren, und/oder
- zwei oder mehr mit einer zweiten Nucleinsäure verbundene erste Bindungsmoleküle,
und/oder das Nachweis-Reagens umfasst:
- zwei oder mehr mit einem zweiten Bindungsmolekül verbundene dritte Nucleinsäuren, und/oder
- zwei oder mehr mit einer dritten Nucleinsäure verbundene zweite Bindungsmoleküle.

Fänger- und/oder Nachweis-Reagens liegen somit als Oligo- und/oder Polymere vor, die jeweils in einem Molekül zwei oder mehr der jeweiligen ersten bzw. zweiten Bindungsmoleküle und/oder jeweils in einem Molekül zwei oder mehr der jeweiligen zweiten bzw. dritten Nucleinsäure umfassen. Mit derartigen oligo- oder polymerisierten Fänger- und/oder Nachweis-Reagenzien kann die Nachweisempfindlichkeit, beispielsweise im Vergleich zu einem herkömmlichen ELISA, nochmals vorteilhaft und in häufig überraschend hohem Ausmaß gesteigert werden.

Zum Herstellen eines Fänger-Reagens-Oligomers (bzw. -Polymers) ist es bevorzugt, ein zumindest zweifach biotinyliertes erstes Bindungsmolekül zu verwenden. Die zweite Nucleinsäure ist bevorzugt kovalent an Streptavidin gebunden, so dass vorzugsweise alle Biotin-Bindungsstellen des Streptavidins erhalten bleiben. Die zweite Nucleinsäure kann stattdessen auch einfach biotinyliert und über eine Biotin-Streptavidin-Bindung an Streptavidin gebunden sein. Die zumindest zweifach biotinylierten ersten Bindungsmoleküle werden mit Streptavidin, an das die zweite Nucleinsäure gebunden ist, zu einem linearen Konjugat und/oder einem Netzwerk verknüpft. Freie Biotin-Bindungsstellen von Streptavidin sollten anschließend durch Zugabe von Biotin abgesättigt werden, um störende Nebenreaktionen zu vermeiden.

Mehrere Streptavidine eines Fänger-Reagens-Oligomers (bzw. -Polymers), die gegebenenfalls mit einer zweiten Nucleinsäure und/oder mit einem ersten Bindungsmolekül verknüpft sind, können auch durch Biotin-Streptavidin-Bindung einer zwei- oder mehrfach biotinylierten Brücken-Nucleinsäure miteinander verbunden sein.

Zum Herstellen von Nachweis-Reagens-Oligomeren (bzw. -Polymeren) werden ein- oder mehrfach, vorzugsweise zwei- bis vierfach, biotinylierte Bindungsmoleküle durch Streptavidin mit ein- oder mehrfach, vorzugsweise zweifach, biotinylierter dritter Nucleinsäure vernetzt. Dabei gilt allgemein, dass mit zunehmendem Biotinylierungsgrad der Bindungsmoleküle die Herstellung des Nachweis-Reagens-Oligomers leichter gelingt. Insbesondere können mit zunehmendem Biotinylierungsgrad allgemein mehr Bindungsmoleküle pro einzelnem Nachweis-Reagens-Oligomer eingebunden werden.

Die dritte Nucleinsäure kann, wie die zweite Nucleinsäure, kovalent an Streptavidin gebunden sein, so dass alle Biotin-Bindungsstellen des Streptavidins erhalten bleiben. Die dritte Nucleinsäure kann jedoch auch mehrfach biotinyliert sein, insbesondere kann sie an beiden 5'-Enden biotinyliert sein, wenn es sich um eine doppesträngige Nucleinsäure handelt, oder auch an einem 5'- und einem 3'-Ende biotinyliert sein. Zur Herstellung des Nachweis-Reagens-Oligomers kann eine mehrfach biotinylierte dritte Nucleinsäure umgesetzt werden a) mit freiem Streptavidin oder auch b) mit Streptavidin, an das eine dritte oder weitere Nucleinsäure (siehe beispielsweise Niemeyer, C.M., Adler, M., Gao, F., Chi, L., *Bioconjugate Chemistry,* "Nanostructured DNA-Protein Networks Consisting of Covalent Oligonucleotide-Streptavidine Conjugates", zur Veröffentlichung angenommen) kovalent gebunden ist. Freie Biotin-Bindungsstellen des Streptavidins sollten wiederum mit Biotin zum Verhindern von Nebenreaktionen gesättigt werden.

Statt einer Biotin-Streptavidin-Kupplung der jeweils beteiligten Substanzen (Bindungsmoleküle, Nucleinsäuren) können diese auch auf andere Weise miteinander verbunden werden. Insbesondere können sie über kovalente Bindungen miteinander verbunden werden. Die Verwendung von Biotin-Streptavidin-Verbindungen ist jedoch bevorzugt, da Biotin und Streptavidin in hoher Reinheit zu geringem Preis und in großer Menge verfügbar sind, die Verknüpfung beider Substanzen mit einer Vielzahl von Stoffen, insbesondere von Nucleinsäuren und Immunglobulinen und Proteinen, leicht möglich ist und weil die Biotin-Streptavidin-Bindung unter üblichen Verfahrensbedingungen sehr stabil und leicht handhabbar ist. Der Umgang mit Biotin-Streptavidin-Systemen ist unter anderem in beschrieben worden von Diamandis, E. P. und T. K. Christopoulos (1991) ("The biotin-(strept)avidin system: principles and applications in biotechnology." *Clin Chem* **37**(5): 625-36).

Bei der Herstellung der im erfindungsgemäßen Nachweisverfahren verwendeten Fänger- bzw. Nachweis-Reagenzien wird man so vorgehen, dass eine vorgewählte Menge des ersten bzw. zweiten Bindungsmoleküls und der jeweils zugehörende(n) Nucleinsäure(n) gemischt und auf geeignete Weise jeweils miteinander verbunden werden. Dabei wird am Ende ein Anteil der jeweiligen Bindungsmoleküle nicht mit jeweils zugehöriger Nucleinsäure verbunden sein.

Es ist nun ein Verfahren bevorzugt, bei dem das Fänger- und/oder das Nachweis-Reagens vor Durchführung der Schritte d) bzw. f) und/oder g) nicht von solchen ersten bzw. zweiten Bindungsmolekülen abgetrennt wird, die nicht mit dem Fänger- bzw. Nachweis-Reagens verbunden sind. Entgegen der Erwartung, dass nicht mit Fänger- bzw. Nachweis-Reagens verbundene Bindungsmoleküle um die Bindungsstellen auf der nachzuweisenden Substanz mit den Fänger- bzw. Nachweis-Reagenzien konkurrieren und die Nachweisgrenze somit unvorteilhaft erhöhen, hat sich herausgestellt, dass auf die Abtrennung verzichtet werden kann, ohne die Nachweisgrenze wesentlich zu beeinträchtigen. Ein solches erfindungsgemäßes Verfahren ist daher in einer vorteilhaft niedrigen Anzahl von Arbeitsschritten und dementsprechend schnell und einfach durchführbar. Es vereinfacht daneben auch die Herstellung der Fänger- und/oder Nachweis-Reagenzien.

Es ist natürlich dessenungeachtet noch immer möglich, das Fänger- und/oder das Nachweisreagens vor Durchführung der Schritte d) bzw. f) und/oder g) aufzureinigen. Auf diese Weise können ungebundene erste bzw. zweite Bindungsmoleküle wiedergewonnen werden. Ferner ermöglicht eine Aufreinigung die Abtrennung der Fänger- und/oder Nachweisreagenzien von im weiteren Verfahrenablauf gegebenenfalls störenden Substanzen. Dies kann zu einer Erniedrigung der Nachweisgrenze führen.

Wenn die nachzuweisende Substanz mehrere Bindungsstellen zum Binden der Bindungsmoleküle besitzt oder sich mehrere nachzuweisende Substanzen verbinden, so dass der Verbund mehrere Bindungsstellen zum Binden der Bindungsmoleküle besitzt, so ist ein Verfahren besonders bevorzugt, bei dem das erste und das zweite Bindungsmolekül im wesentlichen gleich oder identisch sind. Dabei sind zwei Bindungsmoleküle im wesentlichen gleich, wenn sie an gleichen oder einander räumlich überlappenden Stellen an der nachzuweisenden Substanz binden. Dies ist insbesondere der Fall, wenn zumindest die Bindungsmolekül-Regionen, die für die Erkennung der nachzuweisenden Substanz verantwortlich sind, identisch oder einander sehr ähnlich sind.

Obwohl zu erwarten war, dass das erste und das zweite Bindungsmolekül einander jeweils durch Konkurrenz um die Bindungsstellen auf der nachzuweisenden Substanz verdrängen und somit die Nachweisgrenze erhöhen würden, wurde überraschend gefunden, dass diese Konkurrenz keinen nachteiligen Effekt auf die mit dem erfindungsgemäßen Verfahren erzielbare Nachweisgrenze ausübt. Dementsprechend ist ein solches Verfahren vorteilhaft einfach durchführbar, da lediglich eine einzige Bindungsmolekül-Sorte statt deren zwei zur Herstellung sowohl des Fänger- als auch des Nachweis-Reagens benötigt wird. Dies ermöglicht es insbesondere, einen monoclonalen Antikörper und/oder dessen Fragmente als erstes und zweites Bindungsmolekül zu verwenden. Dadurch wird die verhältnismäßig aufwendige Gewinnung eines zweiten monoclonalen Antikörpers umgangen.

Ferner ist ein Verfahren bevorzugt, bei dem die nachzuweisende Substanz in wässriger Lösung Aggregate bildet. Aggregatbildenden Substanzen sind insbesondere lipophile und amphiphile Substanzen. Die Aggregate können in herkömmlichen Nachweisverfahren ausfallen oder diese auf sonstige Weise beeinträchtigen. Insbesondere führt die Aggregatbildung dazu, dass ein Anteil der im günstigsten Fall verfügbaren Bindungsmolekül-Bindungsstellen auf der nachzuweisenden Substanz im Inneren der Aggregate verborgen (maskiert) ist und zur Bindung der Bindungsmoleküle tatsächlich nicht zur Verfügung steht.

Es hat sich jedoch überraschend herausgestellt, dass die Nachweisgrenze eines erfindungsgemäßen Verfahrens für solche Substanzen vorteilhaft niedriger liegt als zu erwarten war. Dies gilt insbesondere für den Fall, dass als erstes und/oder zweites Bindungsmolekül ein Immunglobulin, insbesondere ein IgG-Antikörper, oder eines der eingangs beschriebenen Fragmente eines Immunglobulins verwendet wird.

Besonders bevorzugt ist ein Verfahren, bei dem die nachzuweisende Substanz ein Prionprotein oder ist. Das Prionprotein (PrP) gilt als ein wichtiges, wenn nicht gar als das auslösende übertragbare Agens neurodegenerativer Krankheiten wie Scrapie, Creutzfeldt-Jakob-Krankheit, bovine spongiforme Encephalopathie und vergleichbarer Krankheiten. Ein Übersichtsartikel von J. Collinge ist in *Hum.Mol.Genet*. 1997, 1699 bis 1705, erschienen. Der Ausdruck "prion protein" wurde von S.B. Prusiner, *Science* 1982, 136 bis 144, zur Kennzeichnung des infektiösen Agens der Scrapie-Krankheit verwendet und hat sich seitdem zum Inbegriff einer ganzen Klasse wirkungsmäßig vergleichbarer Proteine entwickelt. Solche Substanzen neigen in wässriger Lösung besonders stark zur Bildung von Aggregaten, die die Bindung von Immunglobulinen (Antikörpern) in herkömmlichen Verfahren stark einschränken und somit nur in unvorteilhaft hoher Konzentration nachweisbar sind. Es hat sich jedoch überraschend herausgestellt, dass der Nachweis von Prionproteinen mit einem erfindungsgemäßen Nachweisverfahren auch bei niedrigen Konzentrationen des Prionproteins möglich ist.

Ebenfalls bevorzugt ist ein Verfahren, bei dem die nachzuweisende Substanz ein Mistel-Lektin ist. Auch Mistel-Lektine neigen in wässriger Lösung stark zur Bildung von Aggregaten, die die Bindung von Immunglobulinen (Antikörpern) in herkömmlichen Verfahren stark einschränken und somit nur in unvorteilhaft hoher Konzentration nachweisbar sind. Es hat sich jedoch überraschend herausgestellt, dass der Nachweis von Mistel-Lektinen mit einem erfindungsgemäßen Nachweisverfahren auch bei niedrigen Konzentrationen des Lektins möglich ist.

Es ist nun in einer Vielzahl von Fällen ein Verfahren bevorzugt, bei dem die Schritte d) bzw. f) und/oder g) im wesentlichen frei von Detergenzien durchgeführt werden. Detergenzien, also chaotrope Stoffe wie beispielsweise Guanidiniumhydrochlorid und grenzflächenaktive Stoffe und Tenside wie beispielsweise TWEEN-20 und TWEEN-80, werden in herkömmlichen Verfahren verwendet, um in wässrigen Lösungen Aggregate lipophiler oder amphiphiler Substanzen zu spalten. Dabei bedeutet im wesentlichen frei von Detergenzien, dass die Konzentration von Detergenzien (grenzflächenaktiven Stoffen und Tensiden) geringer ist als 1 Vol.-%. Vorzugsweise ist die Konzentration sogar geringer als 0,2 Vol.-%, besonders bevorzugt geringer als 0,1 Vol.-%. Die Verwendung von Detergenzien stört häufig die Bindung von Bindungsmolekülen an die nachzuweisende Substanz und wird deshalb in herkömmlichen Verfahren nach Möglichkeit vermieden. Bei stark aggregierenden Substanzen, insbesondere lipophilen oder amphiphilen Substanzen, wird jedoch die Verwendung von Detergenzien als unumgänglich angesehen. Es hat sich nun überraschenderweise herausgestellt, dass bei Verwendung erfindungsgemäßer Fänger- und/oder Nachweis-Reagenzien die Menge an benötigten Detergenzien im Vergleich zu herkömmlichen Verfahren erheblich gesenkt werden kann oder dass sogar auf die Verwendung von Detergenzien völlig verzichtet werden kann. Dies ermöglicht es, eine überraschend niedrige Nachweisgrenze zu erzielen. Hinzu kommt, dass die Durchführung des Nachweisverfahrens durch den Verzicht auf Detergenzien in den Schritten d) und/oder e) vorteilhaft vereinfacht wird.

Die Aufgaben werden ferner durch einen Testassay zum Nachweisen einer oder mehrerer Substanzen in einer wässrigen Lösung gelöst, bei dem mit der wässrigen Lösung mehrere Verfahren einer der zuvor beschriebenen Arten gleichzeitig oder nacheinander durchgeführt werden, wobei sich die Verfahren jeweils voneinander unterscheiden in
a) den ersten und jeweils zugeordneten zweiten Nucleinsäuren und/oder
b) den dritten Nucleinsäuren.

Ein solches Testverfahren ist insbesondere zur Durchführung in einem automatischen Analysegerät geeignet. Insbesondere können solche Verfahren automatisiert werden, um im Mikroliter- und Nanoliterbereich in Mikrotiterplatten und/oder auf Biochips durchgeführt zu werden.

Mithilfe des Verfahrens können auf vorteilhaft einfache Weise mit einer einzelnen Lösung eine oder mehrere darin enthaltene Substanzen nachgewiesen werden. Dabei werden beispielsweise unterschiedliche Bindungsmoleküle zum Nachweis unterschiedlicher Substanzen oder zum Nachweis einer Substanz mit unterschiedlichen Nachweiskinetiken verwendet. Jedem unterschiedlichen ersten und/oder zweiten Bindungsmolekül ist dann jeweils eine spezifische dritte oder jeweils eine spezifische erste und zweite Nucleinsäure zugeordnet:
- Bei Verwendung spezifischer erster und zweiter Nucleinsäure kann in Abhängigkeit vom Immobilisationsort des Nachweiskonjugats (ortsaufgelöst) auf das jeweils zugeordnete erste Bindungsmolekül und gegebenenfalls auf die diesem zugeordnete nachzuweisende Substanz geschlossen werden.
- Bei Verwendung jeweils spezifischer dritter Nucleinsäuren können diese jeweils spezifisch (signalaufgelöst) nachgewiesen werden; damit kann unabhängig vom Immobilisationsort des Nachweiskonjugats zwischen den jeweils zugeordneten zweiten Bindungsmolekülen und gegebenenfalls zwischen den diesen zugeordneten unterschiedlichen Substanzen unterschieden werden.
- Bei Verwendung sowohl spezifischer erster und zweiter Nucleinsäure als auch jeweils spezifischer dritter Nucleinsäuren können sowohl orts- als auch signalaufgelöst dritte Nucleinsäuren nachgewiesen werden; damit können sowohl aufgrund des Immobilisationsorts als auch aufgrund der Art der nachgewiesenen dritten Nucleinsäure auf das jeweilige erste und zweite Bindungsmolekül und gegebenenfalls auf die jeweils zugeordnete Substanz geschlossen werden.

Bevorzugt ist ferner die Verwendung eines Fänger-Reagens, umfassend eine zweite Nucleinsäure und ein mit dieser verbundenes erstes Bindungsmolekül, das an eine nachzuweisende Substanz binden kann, zur Durchführung eines der zuvor beschriebenen erfindungsgemäßen Verfahren. Durch die Verwendung eines solchen Fänger-Reagens mit den Eigenschaften des Fänger-Reagens aus Schritt b) des erfindungsgemäßen Nachweisverfahrens lassen sich die mit der Verwendung eines erfindungsgemäßen Verfahrens verbundenen Vorteile erzielen.

Ferner ist ein Nachweiskonjugat bevorzugt, das erhältlich ist durch ein Verfahren umfassend die Schritte b), c) und d) nach einem der zuvor beschriebenen erfindungsgemäßen Nachweisverfahren. Mit einem solchen Nachweiskonjugat können die oben beschriebenen Vorteile der mit den Substanzen und der Verfahrensdurchführung der Schritte b) bis d) vorteilhaft einfach erreicht werden.

Ferner ist ein Kit zum Herstellen eines solchen Nachweiskonjugats bevorzugt, umfassend
a) ein erstes Bindungsmolekül und gegebenenfalls ein zweites Bindungsmolekül,
b) eine zweite und eine dritte Nucleinsäure,
c) Mittel zum Verbinden des ersten Bindungsmoleküls mit jeweils der zweiten bzw. dritten Nucleinsäure, bzw. Mittel zum Verbinden des ersten Bindungsmoleküls mit der zweiten Nucleinsäure und Mittel zum Verbinden des zweiten Bindungsmoleküls mit der dritten Nucleinsäure.

Mit einer Zusammenstellung von Substanzen in einem solchen erfindungsgemäßen Kit kann ein Nachweiskonjugat der beschriebenen Art vorteilhaft einfach hergestellt werden.

Die Erfindung wird nachfolgend anhand der Figuren und einiger Ausführungsbeispiele näher beschrieben. Es stellen dar:
- Fig. 1:: Schematische Darstellung der Herstellung von Fänger- und Nachweis-Reagens-Oligomeren, Bindung beider Oligomere an eine nachzuweisende Substanz, Immobilisierung des Fänger-Oligomers und Nachweis der dritten Nucleinsäure;
- Fig. 2a:: schematische Struktur des Nachweis-Reagens-Oligomers nach Fig. 1;
- Fig. 2b:: schematische Struktur des Fänger-Reagens-Oligomers nach Fig. 1;
- Fig. 3:: Schematische Darstellung der Bindung von Fänger- und Nachweis-Reagens-Oligomeren an eine aggregierte nachzuweisende Substanz;
- Fig. 4:: Schematische Darstellung des Nachweisschrittes a) im erfindungsgemäßen Verfahren, b) bei herkömmlicher Immuno-PCR, c) bei einem herkömmlichen ELISA;
- Fig. 5:: Ablaufplan der durchgeführten Experimente

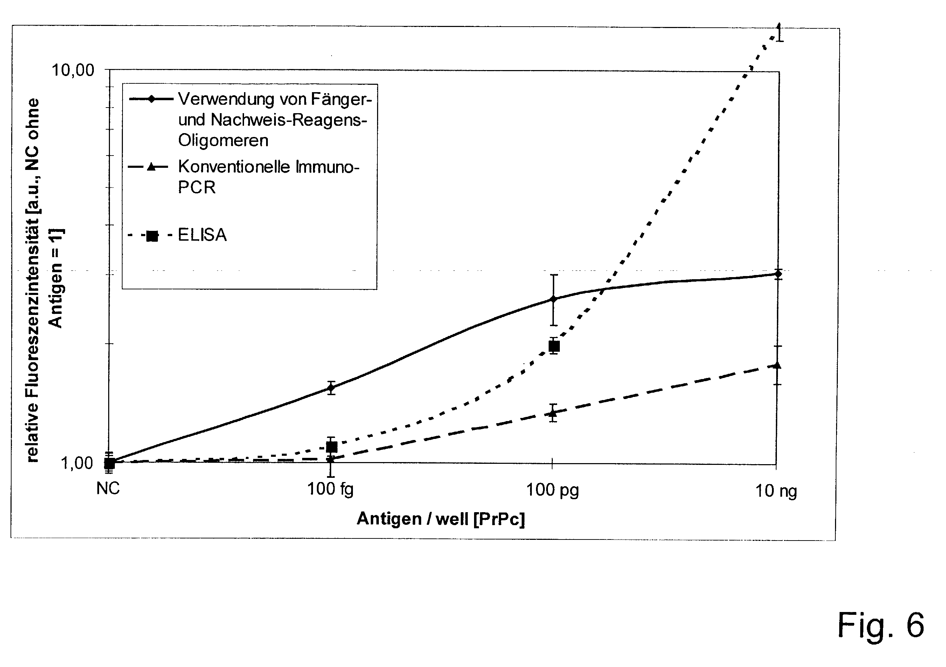

- Fig. 6:: Darstellung der Signalintensitäten gegen die Konzentration der nachzuweisenden Substanz (Antigen) bei verschiedenen Nachweisverfahren

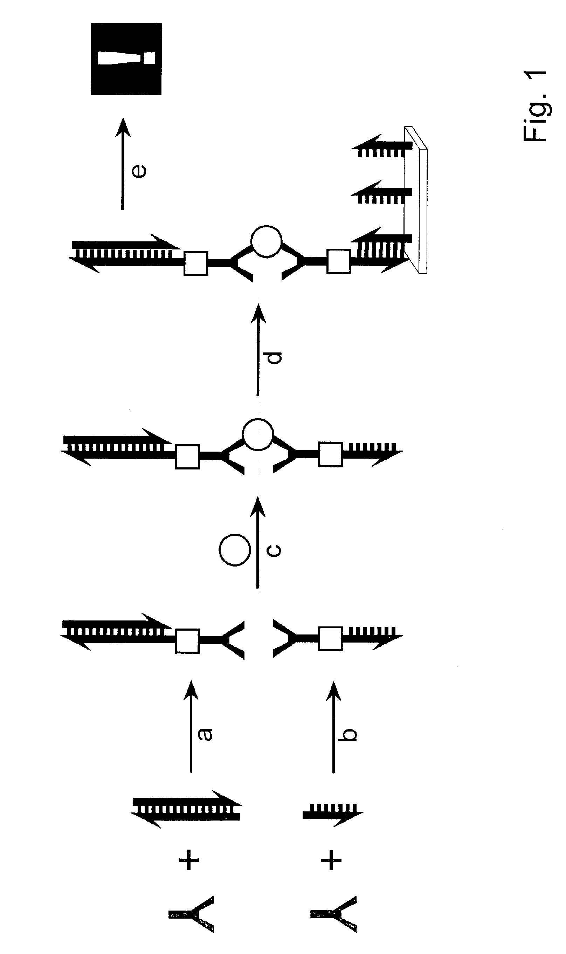

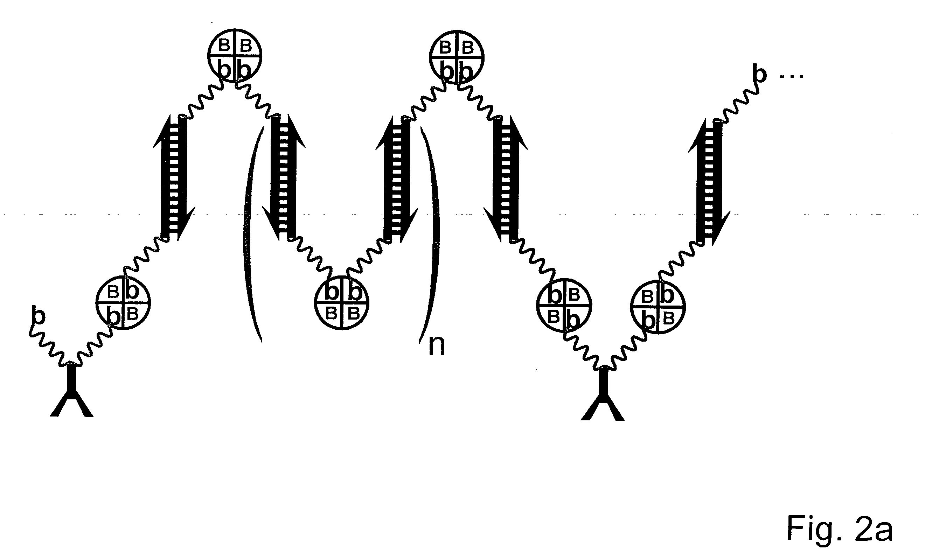

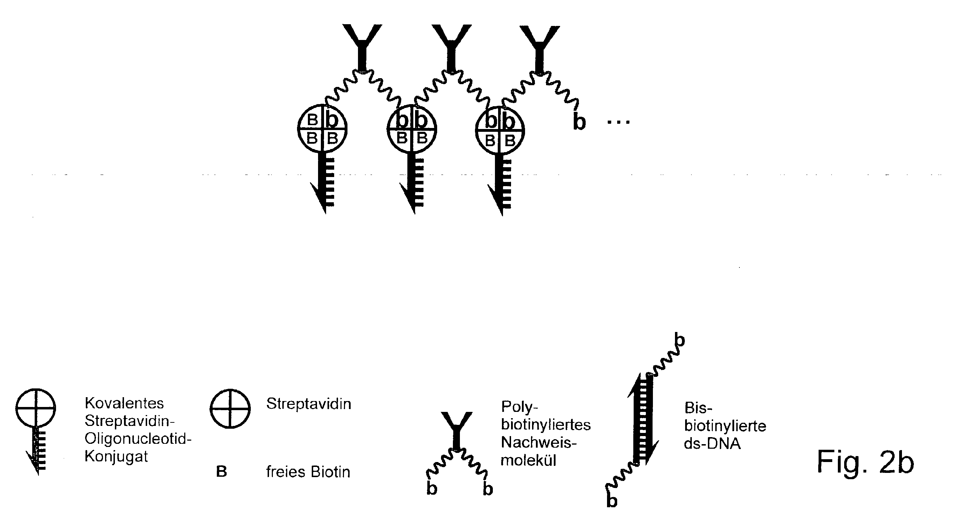

Figur 1 zeigt schematisch den Ablauf des Nachweises unter Aufbau (a - d) des Nachweiskonjugates aus einer Nachweissubstanz, die ein Antigen umfasst (Kreis), einem Nachweis-Reagens-Oligomer und einem Fänger-Reagens-Oligomer sowie die anschließende Signalerzeugung (e):
a.) Synthese des Nachweis-Reagens-Oligomers (hier schematisch als Monomer dargestellt; zur Struktur siehe Fig. 2a) aus Bindungsmolekül (Y-förmig dargestellt) und dritter Nucleinsäure (Doppelpfeil)
b.) Synthese des Fänger-Reagens-Oligomers (hier schematisch als Monomer dargestellt; zur Struktur siehe Fig. 2b) aus Bindungsmolekül (Y-förmig dargestellt) und zweiter Nucleinsäure (einfacher Pfeil mit schematisch angedeuteten Nucleobasen)
c.) Kupplung von Fänger- und Nachweis-Reagens-Oligomer durch Zugabe der Antigen-umfassenden Nachweissubstanz
d.) Immobilisierung des Nachweiskonjugates durch Hybridisierung mit einer ersten Nucleinsäure (einfacher Pfeil mit schematisch angedeuteten Nucleobasen), die an eine Oberfläche gebunden ist. Dabei können die Schritte c.) und d.) gleichzeitig erfolgen.
e.) Nachweis (Ausrufungszeichen) der immobilisierten dritten Nucleinsäure des Nachweis-Reagens-Oligomer.

Im ersten Anwendungsbeispiel wurde der biotinylierte Antikörper 6H4 als Bindungsmolekül, die einzelsträngige Nucleinsäure mit der Oligonucleotid-Sequenz "cA" als erste, "A" als zweite und das doppelt biotinylierte ds-DNA-Fragment "AG" als dritte Nucleinsäure eingesetzt.

Im zweiten Anwendungsbeispiel wurde der biotinylierte Antikörper T2b (gegen rViscumin) als Bindungsmolekül eingesetzt.

Figur 2 zeigt schematisch den Aufbau der in den Anwendungsbeispielen verwendeten Nachweis- (a) und Fänger-Reagens-Oligomere (b). Zur Herstellung des Nachweis-Reagens-Oligomers wurde zunächst ein Aggregat aus tetravalentem STV (Streptavidin, dargestellt als Kreise mit vier Segmenten) und bis-biotinylierter ds-DNA (dargestellt als Doppelpfeile mit einem wellenlinienförmig angedeuteten Linker und Biotin (b)) synthetisiert. Die so erhaltenen Aggregate besitzen unterschiedliche Größe (n: 0 bis ca. 20) und sind in der Abbildung vereinfacht dargestellt, da neben den gezeigten Kettenstrukturen auch Netzwerke mit tri- und tetravalentem STV gebildet werden. Die STV-DNA-Aggregate wurden im Anschluss mit einem polybiotinylierten Bindungsmolekül zu den verwendeten Nachweis-Reagens-Oligomeren weitervernetzt.

Die Darstellung des Fänger-Reagens-Oligomers ist ebenfalls vereinfacht, da auch hier weitere Verzweigungen der gezeigten Kettenstruktur möglich sind.

Beide Oligomere wurden jeweils abschließend mit freiem Biotin (dargestellt durch "B") abgesättigt, um die verbleibenden Biotin-Bindugsstellen des STV zu besetzen und eine direkte Kupplung der Oligomere über STV-Biotin-Interaktion zu unterbinden.

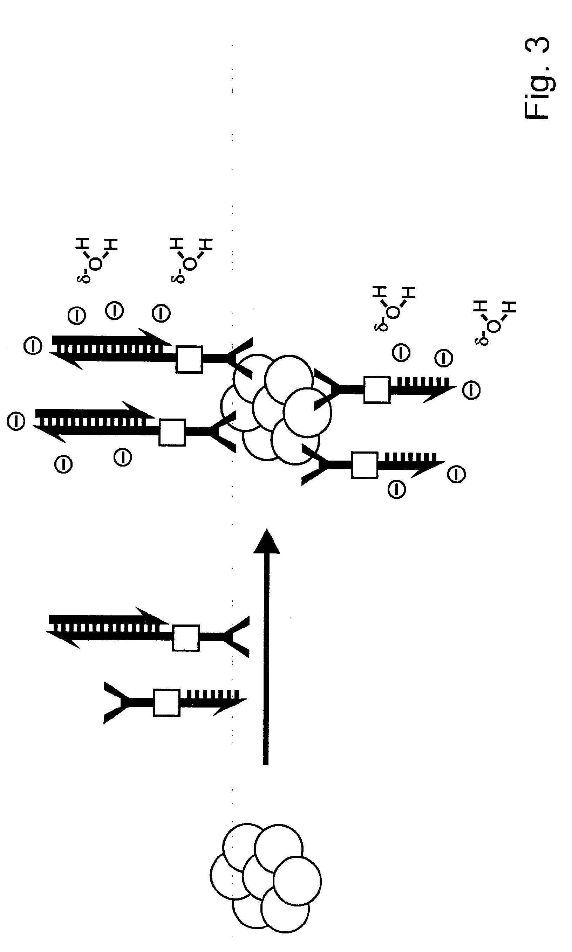

In Figur 3 ist die Bindung mehrerer Fänger- und Nachweis-Reagens-Oligomere mit identischem Bindungsmolekül an ein aggregatbildendes Antigen dargestellt. Dabei trägt wahrscheinlich die negative Partialladung der Nucleinsäuren der Oligomere zur Erhöhung der Löslichkeit des gebildeten Nachweiskonjugates in wässrigen Lösungen bei.

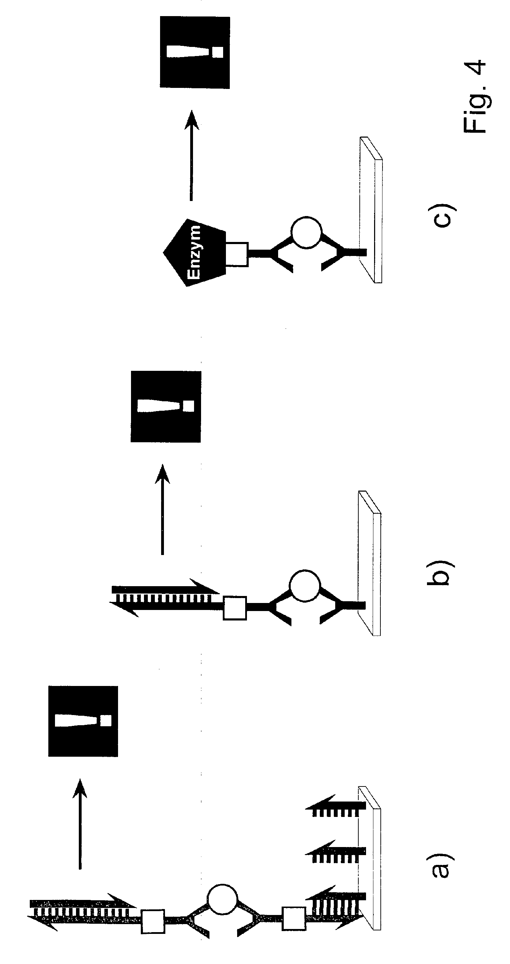

Figur 4 zeigt eine Übersicht der in den Anwendungsbeispielen miteinander verglichenen Nachweis-Techniken:
a.) Antigen-Nachweis mittels Fänger- und Nachweis-Reagens-Oligomeren
b.) Antigen-Nachweis mittels Sandwich Immuno-PCR
c.) Antigen-Nachweis mittels ELISA

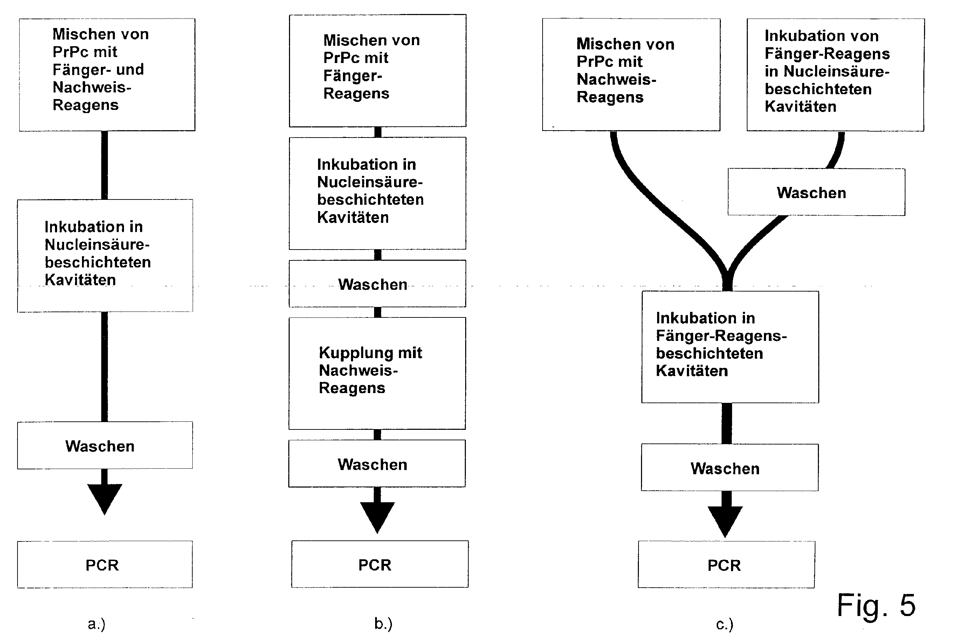

In Figur 5 ist ein Ablaufschema des unterschiedlichen Aufbaus eines Nachweiskonjugates aus Fänger- und Nachweis-Reagens im ersten Anwendungsbeispiel dargestellt:
a.) Einstufige Kupplung (1.4.1.). Es wurde sowohl ungereinigtes als auch gereinigtes Nachweis-Reagens-Oligomer verwendet.
b.) Zweistufige Kupplung unter Inkubation von PrPc mit Fänger-Reagens-Oligomer (1.4.2.)
c.) Zweistufige Kupplung unter Inkubation von PrPc mit Nachweis-Reagens-Oligomer (1.4.3.)

### 1. Ausführungsbeispiel: Nachweis von PrPc

In diesem Anwendungsbeispiel wurden Fänger- und Nachweis-Reagens-Oligomere zum Nachweis von PrPc, der nicht-Krankheits-assoziierten Form des Prion-Proteins, eingesetzt. Der Nachweis wurde mittels der kombinierten Inkubation von PrPc, Fänger-Reagens-Oligomer und Nachweis-Reagens-Oligomer auf einer mit einer ersten Fänger-Nucleinsäure beschichteten Oberfläche und anschließender PCR zur Amplifikation der im Nachweis-Reagens-Oligomer enthaltenen dritten Nucleinsäure durchgeführt (Siehe Abb. 1) Die abschließende Detektion des PCR-Amplifikates erfolgte durch einen PCR-ELISA.

### 1.1. Beschichten von Mikrotiter-Modulen mit der ersten Nucleinsäure

Die nicht-kovalente Verknüpfung der Reaktionsgefäße mit der zur Immobilisierung des Fänger-Reagens-Oligomers benötigten ersten Nucleinsäure erfolgte durch Physisorption des Kupplungsproteins Streptavidin (STV) an die zu funktionalisierende Oberfläche und anschließender Inkubation mit biotinylierter Nucleinsäure (Niemeyer, C. M., L. Boldt, B. Ceyhan und D. Blohm (1999), "DNA-Directed immobilization: efficient, reversible, and siteselective surface binding of proteins by means of covalent DNA-streptavidin conjugates." *Anal Biochem* **268**(1): 54-63).

### 1.1.1. Immobilisierung des STV

Hierzu wurde zunächst eine 0,2 µM Lösung von rekombinantem core-STV (IBA, Göttingen) in PBS-Puffer (200 mM Phosphat (KH₂PO₄ / K₂HPO₄), pH 7) hergestellt. Je Kavität wurden 30 µl dieser Lösung auf Top-Yield Mikrotitermodule (Nunc) aufgetragen, für eine Minute bei 4000 upm in einer Zentrifuge mit Mikrotiterplattenrotor zentrifugiert, und für 72 h bei 4°C inkubiert. Nicht gebundenes STV wurde im Anschluss durch dreimaliges, je dreiminütiges Waschen mit 220 µl TBS-Puffer (20 mM Tris-CI, 150 mM NaCI, pH 7,5) je Kavität entfernt. Danach wurde für mindestens 12h bei 4°C ein Blockierungsschritt gegen unspezifische Wechselwirkungen mit 240 µl MESTBS-Puffer (TBS, 45% Milchpulver (Oxoid), 0,2% NaN₃, 5 mM EDTA, 1 mg/ml DNA MB-grade (Roche)) pro Kavität durchgeführt. Die STV-beschichteten Mikrotitermodule können bis zu eine Woche mit diesem Puffer gelagert werden.

### 1.1.2. Kupplung mit biotinylierter Nucleinsäure

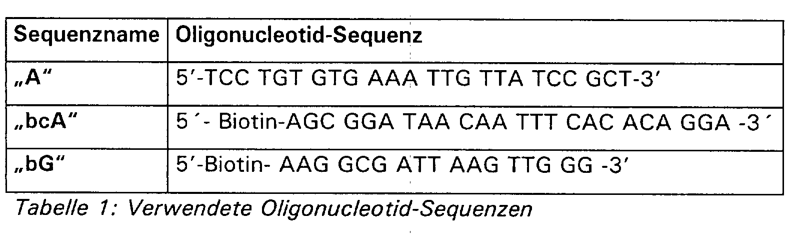

Zur Immobilisierung des Fänger-Reagens-Oligomers, welches die Nucleinsäure-Sequenz "A" (siehe Tabelle 1) enthält, wurde die komplementäre biotinylierte Nucleinsäure "bcA" (Tabelle 1, Interactiva) verwendet. Eine 100 µM Stammlösung von bcA in TE-Puffer (10 mM Tris-CI, 1mM EDTA, pH 7,5), gelagert bei -20°C, wurde unmittelbar vor Gebrauch in TETBS-Puffer (TBS, 5 mM EDTA, 0,05% Tween-20) auf eine Konzentration von 0,24 µM verdünnt.

Die STV-beschichteten und geblockten Mikrotitermodule wurden je zweifach für eine Minute und zweifach für fünf Minuten mit 220 µl TETBS pro Kavität gewaschen, und für 30 Minuten mit 30 µl je Kavität der verdünnten Lösung der biotinylierten Nucleinsäure bcA inkubiert.

Abschließend wurde dreifach für jeweils drei Minuten mit 220 µl je Kavität TETBS-Biotin (TETBS, 800 µM Biotin) gewaschen, um die verbleibende Biotinbindungsfähigkeit der STV-beschichteten Module abzusättigen. Die solcherart vorbereiteten Mikrotitermodule sind für 12 h bei 4°C mit 100 µl je Kavität TETBS-Biotin lagerbar.

### 1.2. Herstellung des Fänger-Reagens-Oligomers

### 1.2.1 Verwendeter biotinylierter Antikörper

Als Bindungsmoleküle zur Synthese von Nachweis- sowie Fänger-Reagens-Oligomeren stehen zur Zeit eine Reihe möglicher Antikörper zur Verfügung, die zum Teil bereits kommerziell erhältlich sind (z.B. 6H4, 5369, 6664, siehe Tabelle 2).

Im folgenden Anwendungsbeispiel wurde der kommerziell erhältliche Antikörper 6H4 (Prionics, Produkt-Nr. 01-010) verwendet.

Für die Kupplung des Antikörpers mit den Nucleinsäuren des Nachweis- sowie des Fänger-Reagens-Oligomers wurde das Biotin/STV-System verwendet (Diamandis and Christopoulos 1991). Der kommerziell nur ohne Biotinylierung erhältliche Antikörper wurde daher nach einem Standardverfahren mit Biotin gekuppelt (Meier, T. und F. Fahrenholz (1996), "A laboratory Guide to Biotin-Labeling in Biomolecule Analysis." *BioMethods* **7** (Birkhäuser Verlag, Basel), 9-13): Zu einem Moläquivalent Antikörper erfolgte die Zugabe von 10 Moläquivalenten Sulfo-NHS-Biotin (Sigma) und anschließende Reinigung über NAP-10-Sephardex-Säulen (Pharmacia).

Der so erhaltene biotinylierte Antikörper wurde für die Synthese des Nachweis- sowie des Fänger-Reagens verwendet.

**Tabelle 2:**

| *Antikörper zum Nachweis von PrP* | | | |
|---|---|---|---|
| **Antikörper** | **Typ** | **Spezies** | **Quelle** |
| **6H4** | monoclonal | Maus | Prionics |
| **34C9** | monoclonal | Maus | Prionics |
| **3F4** | monoclonal | Maus | veröffentlicht von Kascsak, R. J., R. Rubenstein, P. A. Merz, M. Tonna-DeMasi, R. Fersko, R. I. Carp, H. M. Wisniewski und H. Diringer (1987), "Mouse polyclonal and monoclonal antibody to scrapie-associated fibril proteins." *J Virol* **61**(12): 3688-93 |
| **5369** | polyclonal | Ziege | Biogenesis Ltd. |
| **6664** | polyclonal | Ziege | Abcam |

### 1.2.2. Kovalentes Aggregat aus Streptavidin und zweiter Nucleinsäure

Zur Kupplung des biotinylierten Antikörpers mit der Nucleinsäure-Sequenz "A" (siehe 1.1.) wurde zunächst ein kovalentes Konjugat "HA" aus STV und einer thiolierten Nucleinsäure "A" hergestellt. Die Synthese dieses Konjugats unter Verwendung eines heterobifunktionellen chemischen Crosslinkers ist in der Literatur ausführlich beschrieben (Niemeyer, C. M., T. Sano, C. L. Smith und C. R. Cantor (1994), "Oligonucleotide-directed self-assembly of proteins: semisynthetic DNA-streptavidin hybrid molecules as connectors for the generation of macroscopic arrays and the construction of supramolecular bioconjugates." *Nucleic Acids Res* **22**(25): 5530-9). Kurzgefasst wurde zunächst in ein kommerziell erhältliches 5'-Amino-modifiziertes Oligonucleotid (Interactiva) eine Thiol-Gruppe eingeführt und diese im Folgeschritt mittels Sulfo-SMCC (Pierce) mit einer Maleimid-aktivierten primären Amino-Gruppe des STV verknüpft. Das nach anschließender lonenaustausch-Chromatographie-Reinigung auf einer FPLC (Pharmacia) erhaltene gereinigte Produkt HA kann gewöhnlich bei 4°C mehrere Monate in TE gelagert werden.

### 1.2.3. Kupplung des Nachweis-Antikörpers mit der zweiten Nucleinsäure

Zur Herstellung des Fänger-Reagens-Oligomers (Abb. 1, b) wurden vier Picomol des Konjugates HA mit einer äquimolaren Menge des biotinylierten Antikörpers in einem Volumen von 8 µl TE-Puffer für 20 min bei Raumtemperatur unter orbitalem Schütteln inkubiert.

Ein Mikroliter dieser Lösung wurde im Anschluss in 500 µl RDB (9 Teile TETBS + 1 Teil MESTBS) aufgenommen. Durch Zugabe eines Mikroliters TETBS-Biotin und Inkubation für 5 min bei Raumtemperatur unter orbitalem Schütteln wurden die verbleibenden Biotinbindungsstellen des Fänger-Reagens-Oligomers abgesättigt. Die Herstellung des Fänger-Reagens-Oligomers erfolgte erst unmittelbar vor Verwendung, die erhaltene Lösung "FRO" kann für 30 min auf Eis gelagert werden. Die Lösung wurde nicht weiter gereinigt oder von unumgesetzten Edukten befreit. Die Struktur des erhaltenen Oligomers ist schematisch in Fig. 2 b gezeigt.

### 1.3.1. Herstellung des Nachweis-Reagens-Oligomers ohne Aufreinigung

Für die Verknüpfung des biotinylierten Antikörpers mit der im PCR-Nachweis als Templat dienenden dritten Nucleinsäure wurde ein in der Literatur beschriebenes selbst-assembliertes Aggregat aus doppelt biotinylierter Nucleinsäure, tetravalentem Streptavidin und biotinyliertem Antikörper eingesetzt (Niemeyer, C. M., M. Adler, B. Pignataro, S. Lenhert, S. Gao, L. Chi, H. Fuchs und D. Blohm (1999), "Self-assembly of DNA-streptavidin nanostructures and their use as reagents in immuno-PCR." *Nucleic Acids Res* **27**(23): 4553-61). Als Nucleinsäure wurde dabei das ebenfalls in der Literatur (Niemeyer, Adler et al. 1999) beschriebene DNA-Fragment "AG" verwendet, ein doppelt 5'-biotinylierter Doppelstrang (ds) mit 169 Basenpaare Länge. Diese Nucleinsäure ist mittels präparativer PCR durch Einsatz der beiden biotinylierten Primer bcA (Tabelle 1, Interactiva) und bG (Tabelle 1, Interactiva) aus der kommerziell erhältlichen Phagen-DNA M13mp18 (New England Biolabs) herstellbar.

Zur Herstellung des Nachweis-Reagens-Oligomers (Abb 1, a) wurde in einem Volumen von 10 µl TE zu einer vorgelegten Menge von 2,5 pmol STV (IBA, siehe 1.1.) ein halbes Moläquivalent von 1,25 pmol der doppelt biotinylierten DNA "AG" gegeben und für 15 min bei Raumtemperatur unter orbitalem Schütteln inkubiert. Zwei Microliter der Lösung des STV-DNA-Aggregats wurden im Anschluss mit 0,25 pmol biotinyliertem Antikörper in einem Volumen von 5 µl TE für 20 min bei Raumtemperatur unter orbitalem Schütteln inkubiert. Zwei Microliter dieser Lösung des Antikörper-STV-DNA-Aggregats wurden in 500 µl RDB aufgenommen. Durch Zugabe eines Mikroliters TETBS-Biotin und Inkubation für 5 min bei Raumtemperatur unter orbitalem Schütteln wurden die verbleibenden Biotinbindungsstellen des Nachweis-Reagens-Oligomers abgesättigt. Die Herstellung des Nachweis-Reagens-Oligomers erfolgt erst unmittelbar vor Verwendung, die so erhaltene Lösung "DRO-1" ("Detektionsreagens-Oligomer") kann für 30 min auf Eis gelagert werden und wurde nicht weiter gereinigt oder von unumgesetzten Edukten befreit. Die Struktur des erhaltenen Oligomers ist schematisch in Abb. 2a gezeigt.

### 1.3.2. Herstellung des Nachweis-Reagens-Oligomers für mehrfache Assays

In einer alternativen Vorgehensweise wurde das Nachweis-Reagens-Oligomer in größerer Menge hergestellt, gereinigt und gelagert.

Zur Herstellung des Nachweis-Reagens-Oligomers wurden 80 pmol des biotinylierten Antikörpers 6H4 wie in Niemeyer, Adler et al. (1999) beschrieben eingesetzt. Nach Aufnahme von 1 µl des gereinigten Reagens-Oligomers in 500 µl RDB und Zugabe von 1 µl TBS-Biotin wird die Lösung "DRO-2" erhalten.

### 1.4. Kupplung mit PrPc

Als Antigen wurde in diesem Anwendungsbeispiel das kommerziell erhältliche bovine PrPc (Prionics, Produkt-Nr. 03-011) verwendet.

Zur Untersuchung der Effizienz des Assays wurde PrPc aus reinem Puffer nachgewiesen. Um das lipophile Protein PrPc in Lösung bzw. Suspension zu halten, wurde hierbei ein schwach detergenzhaltiger Puffer (s. nächster Absatz) verwendet. Es erfolgte jedoch kein gesonderter Desintegrationsschritt.

Eine Verdünnungsreihe mit den Konzentrationen 3,3 µg/ml, 33 ng/ml und 33 pg/ml PrPc sowie eine Negativkontrolle ohne PrPc wurde in PIP (0,1 M NaCl, 0,05 M Tris, 0,05% DOC (deoxycholic acid, Sigma), 0,05% Nonidet P40 (Sigma)) in silikonisierten 1,5 ml Reaktionsgefäßen (Biozym) vorbereitet.

Alternativ wurde der Detergenz-freie Puffer TBS verwendet.

### 1.4.1. Einstufige Kupplung

Für die Kupplung des PrPc wurden zunächst jeweils 35 µl "FRO" und 35 µl "DRO-1" oder "DRO-2" in silikonisierten 1,5 ml Reaktionsgefäßen (Biozym) vorgelegt. Zu diesem Gesamtvolumen von 70 µl wurden je 10 µl der PrPc-Lösungen gegeben und gut durchmischt. Der Ansatz wurde in einer Doppelbestimmung von 30 µl je Kavität sofort auf die Nucleotid-beschichteten Mikrotitermodule (siehe 1.1.) gegeben und für eine Stunde bei 37°C unter orbitalem Schütteln inkubiert. Dies entspricht einer gleichzeitigen Durchführung der in Fig. 1 c und d gezeigten Schritte (Fig. 5 a).

### 1.4.2. Zweistufige Kupplung unter Inkubation von PrPc mit Fänger-Reagens-Oligomer

In einer alternativen Vorgehensweise wurde zunächst je 10 µl PrPc-Lösung in 70 µl "FRO" aufgenommen. Auch diese Mischung wurde direkt ohne vorhergehende Inkubation zu je 30 µl / Kavität in Nucleinsäure-beschichtete Mikrotitermodule überführt und dort für eine Stunde bei 37°C inkubiert. Nach vierfachen Waschen der Module mit 220 µl / Kavität TETBS für je zwei mal eine Minute und zwei mal fünf Minuten unter orbitalem Schütteln erfolgte in einem zweiten Schritt die Inkubation mit 30 µl / Kavität der Lösung "DRO-1" für ebenfalls eine Stunde bei 37°C (Fig. 5 b).

### 1.4.3. Zweistufige Kupplung unter Inkubation von PrPc mit Nachweis-Reagens-Oligomer

Eine dritte Vorgehensweise begann mit der einstündigen Inkubation von 30 µl der Lösung "FRO" bei 37°C in Nucleinsäure-beschichteten Mikrotitermodulen. Parallel zu diesem Schritt wurden je 10 µl der PrPc-Lösung mit 70 µl der Lösung "DRO-1" für eine Stunde bei 37°C inkubiert. Nach einem wie unter 1.4.2. beschriebenen Waschschritt der Module erfolgte der Transfer von je 30 µl / Kavität der Lösung von PrPc und Nachweis-Reagens-Oligomer in die mit Nucleinsäure und Fänger-Reagens-Oligomer beschichteten Mikrotitermodule. Im Anschluss erfolgte eine einstündige Inkubation der Lösung von PrPc und DRO-1 in den Mikrotitermodulen bei 37°C unter orbitalem Schütteln (Fig. 5 c).

### 1.4.4. Kontroll-Immuno-PCR ohne Verwendung des Fänger-Reagens-Oligomers

Zur Überprüfung der Effektivität der verfahrensgemäßen Immobilisierung mittels Nucleinsäure-enthaltender Fänger-Reagens-Oligomere wurde eine konventionelle Sandwich-Immuno-PCR mit festphasenimmobilisierten Fänger-Antikörpern durchgeführt (siehe Fig. 4 b). Hierzu wurden Top-Yield Mikrotitermodule (Nunc) mit 30 µl je Kavität einer Lösung von 2,5 µg/ml des jeweiligen nicht-biotinylierten Antikörpers in RB-Puffer (50 mM Borsäure / NaOH, pH 9,5) für 12 Stunden bei 4°C inkubiert. Der Blockierungsschritt der Antikörper-beschichteten Module wurde entsprechend dem unter 1.1. geschilderten Blockierungsschritt für STV-beschichtete Module durchgeführt. Nach dem auf die Blockierung folgenden vierfachen Waschschritt mit TETBS wurden die Module für eine Stunde bei 37°C unter orbitalem Schütteln mit 30 µl / Kavität der unter 1.4.3. beschriebenen Lösung von PrPc und DRO-1 inkubiert.

### 1.5. Polymerase-Kettenreaktion (PCR).

Zum Nachweis der im Nachweis-Reagens-Oligomer enthaltenen dritten Nucleinsäure "AG" (siehe Abb. 1 e) wurde eine enzymatische Vervielfältigung mittels PCR durchgeführt (Niemeyer, Adler et al. 1999). Vor diesem Amplifikationsschritt wurde zunächst zweifach für je eine Minute und fünffach für je fünf Minuten mit 220 µl TETBS je Kavität unter orbitalem Schütteln bei Raumtemperatur gewaschen. Abschließend wurde noch zweifach für je eine Minute mit 220 µl TBS je Kavität gewaschen, um das PCR-hemmende EDTA des TETBS-Puffers aus den Kavitäten zu entfernen.

Die Module wurden mit Klebefolie (Plate Sealers, Dynal) verschlossen. Die Durchführung der PCR erfolgte in einem Gene Amplifying System 9600 (Perkin Elmer) oder alternativ in einem PTC-200 (MJ-Research) Thermalzykler mit 96-well Heizblock und aktiviertem Heizdeckel. Die PCR wurde wie in Niemeyer, Adler et al. (1997, 1999) beschrieben durchgeführt.

Der PCR-Mastermix enthielt biotinylierte Primer und Digoxigenin-dUTP, die durch eine zweifache Funktionalisierung des PCR-Amplifikates eine Detektion des erzeugten Nucleinsäure mittels PCR-ELISA erlauben. Dieses in der Literatur (Niemeyer, C. M., M. Adler und D. Blohm (1997), "Fluorometric Polymerase Chain Reaction (PCR) Enzyme-Linked Immunosorbent Assay for Quantification of Immuno-PCR Products in Microplates." *Anal Biochem* **246**(1): 140-5) beschriebene Verfahren ermöglichte über die Kupplung des biotinylierten PCR-Amplifikates an STV-beschichtete Mikrotitermodulen und anschließende Verknüpfung mit einem Anti-Digoxigenin Antikörper-Enzymkonjugat (Roche) einen empfindlichen, quantifizierbaren und reproduzierbaren enzymatischen Nachweis des Amplifikates. Dabei wurde für eine maximale Sensitivität des Assays das Fluoreszenz-generierenden Substrat AttoPhos™ (Roche) der alkalischen Phosphatase eingesetzt und mittels eines VICTOR 1420 Multilabelcounters (Wallac) das Fluoreszenzsignal vermessen.

### 1.6. Kontroll-ELISA

Um die Nachweisgrenze konventioneller Verfahren zum Nachweis des PrPc mit den verwendeten Reagenzien zu Vergleichszwecken zu ermitteln wurde ein ELISA des Prion-Proteins PrPc durchgeführt (Siehe Fig. 4 c).

Es wurden zunächst drei Stammlösungen von PrPc in TBS mit Konzentrationen von 3,3 µg/ml, 33 ng/ml und 33 pg/ml sowie eine Negativkontrolle ohne PrPc vorbereitet. Im Anschluss wurden je 10 µl der PrPc-Lösungen in je 70 µl TBS-Puffer mit 2 ng/ml des biotinylierten Detektionsantikörpers aufgenommen und für eine Stunde bei 37°C in einem Eppendorf-Thermomixer bei 150 rpm inkubiert.

Mikrotitermodule, die gemäß 1.4.4. mit Fänger-Antikörper beschichtet, geblockt und mit TETBS gewaschen waren, wurden mit 30 µl je Kavität der PrPc-haltigen Lösung für eine Stunde bei 37°C unter orbitalem Schütteln inkubiert. Nach einem weiteren Waschschritt mit TETBS (siehe 1.4.2.) wurden die Module mit 30 µl / Kavität einer 1:5000 Verdünnung eines STV-Alkalische-Phosphatase-Konjugates (1000 U/ml vor Verdünnung, Roche) in TBS für eine Stunde bei Raumtemperatur unter orbitalem Schütteln inkubiert. Im Anschluss wurde ein weiterer TETBS-Waschschritt durchgeführt, bevor abschließend dreifach für je drei Minuten mit 220 µl / Kavität TBS gewaschen wurde. Nach Zugabe von 50 µl / Kavität des Fluoreszenz-generierenden Substrates AttoPhos™ (Roche) wurde die erhaltene Fluoreszenz mit einem VICTOR-Multilabelcounter (Wallac) gemessen.

### 1.7. Ergebnisse

Es konnte gezeigt werden, dass mit allen durchgeführten Vorgehensweisen zum Aufbau des Nachweiskonjugates (Fig. 5) der Nachweis von ca. 100 Femtogramm PrPc (ca. 4,1 amol/µl) möglich war (siehe Tabelle 3). Bei einer Nachweisgrenze konventioneller ELISA-Verfahren (siehe 1.6. und Fig. 4 c) zum Nachweis des PrP von ca. 10 pg unter analogen Bedingungen entspricht dies einer Verbesserung der Nachweisempfindlichkeit um einen Faktor 100. Im Vergleich zu einer konventionellen Immuno-PCR (1.4.4., Fig. 4 b) wurde durch die Kombination von Fänger- und Nachweis-Reagens-Oligomer in diesem Verfahren ein deutlicher Gewinn an Sensitivität erzielt (siehe Tabelle 3, b und c).

**Tabelle 3:**

| *Ergebnisse des 1. Ausführungsbeispiels: Nachweis von PrPc. Angegeben sind jeweils relative Signalintensitäten, bezogen auf eine Negativkontrolle ohne Antigen = 1. Der mittlere Fehler der Werte liegt bei ca. 15%. Die angegebene PrPc-Menge bezieht sich jeweils auf 30 µl Probenvolumen in den Mikrotitermodulen.* | | | |
|---|---|---|---|
| Experiment | **0,1 ng PrPc** | **0,1 pg PrPc** | **Kein PrPc** |
| **a.)** ELISA (1.6.) | 2 | 1,1 | 1 |
| **b.)** Konventionelle IPCR (1.4.4.) | 1,6 | 1,2 | 1 |
| **c.)** Verwendung von Fänger- und Nachweis-Reagens-Oligomer, einstufige Inkubation (1.4.1.) | **2,6** | **1,55** | **1** |
| **d.)** 1.4.1. bei Verwendung einer nicht-komplementären festphasen-gebundenen ersten Nucleinsäure | 1 | 1 | 1 |
| **e.)** 1.4.1. bei Verwendung eines Detergenz-freien Puffers ("TBS") | 2,5 | 1,5 | 1 |
| **f.)** Vorinkubation von PrPc und Fänger-Reagens-Oligomer (1.4.2) | 1,6 | 1,3 | 1 |
| **g.)** Vorinkubation von PrPc und Nachweis-Reagens-Oligomer (1.4.3.) | 1,5 | 1,3 | 1 |
| **h.)** 1.4.1 mit vorhergehender Aufreinigung des Nachweis-Reagens-Oligomers | 3,8 | 1,4 | 1 |

Es konnte ebenso gezeigt werden, dass das Verfahren bei Inkubation von PrP, Fänger- und Nachweis-Reagens-Oligomer in völlig Detergenz-freiem TBS-Puffer ohne Einbußen an Sensitivität durchführbar ist (siehe Tabelle 3, e). Der Vergleich zwischen schrittweiser Inkubation der einzelnen Komponenten des Nachweissystems (1.4.2. bzw. 1.4.3., Fig. 5, b und c) und einer einstufigen Inkubation (1.4.1., Fig. 5 a) ergab die besten Signal/ Hintergrundwerte bei einer einstufigen Vorgehensweise (siehe Tabelle 3, c). Ein gereinigtes Nachweis-Reagens-Oligomer (siehe Tabelle 3, h) erwies sich ebenfalls als funktional. Die Immobilisierung erfolgte ortsspezifisch durch die Hybridisierung mit der oberflächengebundenen ersten Nucleinsäure, bei Verwendung einer nicht-komplementären ersten Nucleinsäure als Negativkontrolle wurde kein Signal erhalten (siehe Tabelle 3, d).

### 2. Ausführungsbeispiel: Nachweis von rekombinantem Mistel-Lektin (rViscumin)

rViscumin, ein rekombinant aus den genetischen Informationen des Mistel-Lektins abgeleiteter neuer pharmakologischer Wirkstoff, hat sich im Tierversuch auch in sehr geringen Dosierungen als hochwirksames AntiTumormittel erwiesen (siehe dazu auch Eck, J., M. Langer, B. Mockel, A. Baur, M. Rothe, H. Zinke und H. Lentzen (1999), "Cloning of the mistletoe lectin gene and characterization of the recombinant A-chain." *Eur J Biochem* **264**(3): 775-84 sowie Eck, J., M. Langer, B. Mockel, K. Witthohn, H. Zinke und H. Lentzen (1999), "Characterization of recombinant and plant-derived mistletoe lectin and their B-chains." *Eur J Biochem* **265**(2): 788-97). Zur weitergehenden Untersuchung insbesondere der pharmakokinetischen und toxikokinetischen Eigenschaften des rViscumins ist ein extrem sensitives Nachweisverfahren von großem Interesse.

Für den Nachweis des rViscumins (Viscum AG), wurden zusätzlich zu Nucleinsäure beschichteten Mikrotitermodulen (siehe 1.1.) Fänger-Reagens-Oligomere und Nachweis-Reagens-Oligomere mit dem biotinylierten polyclonalen Kaninchen-anti-rViscum Antikörper T2b ("Tier 2", #2758g, 1,2 mg/ml, ca. 8 pmol/µl, 2,7 mol Biotin / 1 mol Antikörper, BRAIN AG) eingesetzt.

### 2.1. Herstellung des Fänger-Reagens-Oligomers

Die Herstellung des Fänger-Reagens-Oligomers (Abb. 1 b und 2 b) für rViscumin erfolgte entsprechend der Vorgehensweise für PrPc unter Verwendung von T2b als biotinyliertem Antikörper.

### 2.2. Herstellung und Reinigung des Nachweis-Reagens-Oligomers

Die Herstellung eines gereinigten Nachweis-Reagens-Oligomers (Abb. 1 a und 2 a) wurde entsprechend 1.3.2. durchgeführt. Dabei wurden 75 pmol T2b anstelle des Anti-PrPc-Antikörpers eingesetzt.

### 2.3. Kupplung mit rViscumin

Das Antigen rViscumin (Viscum AG) wurde mit einer Konzentration von 2 ng/µl in Aliquoten zu je 10 µl bei -80°C gelagert und erst unmittelbar vor Gebrauch aufgetaut. rViscumin-haltige Lösungen sollten nicht bei Raumtemperatur und nicht länger als 20 min auf Eis gelagert werden. Die Verdünnung des rViscumins auf Konzentrationen von 100 ng/ml, 100 pg/ml und 1 pg/ml erfolgte in BISEKO, einem virusinaktivierten standardisierten Humanserum der Firma Biotest, um die Assaybedingungen eines rViscumin-Nachweises aus Patientenserum zu simulieren. Als Negativkontrolle wurde reines BISEKO verwendet.

In silikonisierten 1,5 ml Reaktionsgefäßen wurde zu einem Gemisch von jeweils 35µl des T2b-enthaltenden Fänger- und Nachweis-Reagens-Oligomers wurden je 10 µl der Lösungen von rViscumin in BISEKO gegeben. Die erhaltenen Gemische wurden sofort in Nucleinsäure-beschichtete Mikrotitermodule (siehe 1.1) überführt und für 30 min bei Raumtemperatur unter orbitalem Schütteln inkubiert. Als Puffer zur Verdünnung des Fängerund Nachweis-Reagens-Oligomers wurde RDB verwendet. Die weitere Durchführung des Assays entspricht 1.5.

### 2.4. Kontrollexperimente

Entsprechend 1.4.4. und 1.6. wurde eine konventionelle IPCR sowie ein ELISA als Kontrollexperiment durchgeführt. Dabei wurden Mikrotitermodule verwendet, die mit 30 µl je Kavität einer 5 µg/ml Lösung des nicht-biotinylierten Antikörpers "T2" ("Tier 2", #2578g, BRAIN AG) beschichtet worden sind. Die Inkubationsschritte erfolgten jeweils bei Raumtemperatur.

### 2.5. Ergebnisse

Die Funktionalität von Fänger- und Nachweis-Reagens-Oligomer konnte in diesem Anwendungsbeispiel auch in einer biologischen Matrix (BISEKO) gezeigt werden (siehe Tabelle 4).

**Tabelle 4:**

| *Nachweis von rViscumin mittels Fänger- und Nachweis-Reagens- Oligomeren. Angegeben sind jeweils relative Signalintensitäten, bezogen auf eine Negativkontrolle ohne Antigen = 1. Der mittlere Fehler der Werte liegt bei ca. 15%.* | | | |
|---|---|---|---|
| Experiment | **100 pg/ml rViscumin** | **1 pg/ml rViscumin** | **Kein rViscumin** |
| a.) ELISA (2.4.) | 1,5 | 1 | 1 |
| b.) Konventionelle IPCR (2.4.) | 3,0 | 1,5 | 1 |
| c.) Verwendung von Fänger- und Nachweis-Reagens-Oligomer, einstufige Inkubation (2.3.) | **1,9** | **1,5** | **1** |

## Patentansprüche

1. Verfahren zum Nachweis einer Substanz in wässriger Lösung, umfassend die Schritte:
a) Bereitstellen einer Oberfläche mit einer damit verbundenen ersten Nucleinsäure,
b) Bereitstellen eines Fänger-Reagens, umfassend eine zweite Nucleinsäure, die mit der ersten Nucleinsäure hybridisieren kann, und ein erstes Bindungsmolekül, das an die nachzuweisende Substanz binden kann, wobei die zweite Nucleinsäure und das erste Bindungsmolekül miteinander verbunden sind,
c) Bereitstellen eines Nachweis-Reagens, umfassend ein zweites Bindungsmolekül, das an die nachzuweisende Substanz binden kann, und eine dritte Nucleinsäure, die mit dem zweiten Bindungsmolekül verbunden ist,
d) Zusammenbringen der mit der ersten Nucleinsäure verbundenen Oberfläche, des Fänger-Reagens, des Nachweis-Reagens und einer wässrigen Lösung der nachzuweisenden Substanz, um ein Nachweiskonjugat aus Fänger-Reagens, nachzuweisender Substanz und Nachweis-Reagens zu bilden und dieses durch Hybridisieren von erster und zweiter Nucleinsäure an die Oberfläche zu binden und/oder an der Oberfläche zu bilden,
e) Nachweisen von dritter Nucleinsäure, soweit sie in einem an die Oberfläche gebundenen Nachweiskonjugat vorliegt.

2. Verfahren nach Anspruch 1, bei dem Schritt d) wie folgt durchgeführt wird:
f) Zusammenbringen des Fänger-Reagens, des Nachweis-Reagens und der nachzuweisenden Substanz zum Herstellen eines Nachweiskonjugats aus Fänger-Reagens, nachzuweisender Substanz und Nachweis-Reagens, und anschließend
g) Zusammenbringen der mit der ersten Nucleinsäure verbundenen Oberfläche mit dem Nachweiskonjugat, um das Nachweiskonjugat durch Hybridisieren von erster und zweiter Nucleinsäure an die Oberfläche zu binden.

3. Verfahren nach einem der vorherigen Ansprüche,
**dadurch gekennzeichnet, dass** zum Nachweisen der dritten Nucleinsäure diese amplifiziert wird.

4. Verfahren nach einem der vorherigen Ansprüche,
**dadurch gekennzeichnet, dass** das Fänger-Reagens umfasst:
- zwei oder mehr mit einem ersten Bindungsmolekül verbundene zweite Nucleinsäuren, und/oder
- zwei oder mehr mit einer zweiten Nucleinsäure verbundene erste Bindungsmoleküle,
und/oder das Nachweis-Reagens umfasst:
- zwei oder mehr mit einem zweiten Bindungsmolekül verbundene dritte Nucleinsäuren, und/oder
- zwei oder mehr mit einer dritten Nucleinsäure verbundene zweite Bindungsmoleküle.

5. Verfahren nach einem der vorherigen Ansprüche,
**dadurch gekennzeichnet, dass** das Fänger- und/oder das Nachweis-Reagens vor Durchführung von Schritt d) bzw. f) und/oder g) nicht von solchen ersten bzw. zweiten Bindungsmolekülen abgetrennt wird, die nicht mit Fänger- bzw. Nachweis-Reagens verbunden sind.

6. Verfahren nach einem der vorherigen Ansprüche,
**dadurch gekennzeichnet, dass** das erste und das zweite Bindungsmolekül im wesentlichen gleich sind.

7. Verfahren nach einem der vorherigen Ansprüche,
**dadurch gekennzeichnet, dass** die nachzuweisende Substanz in wässriger Lösung Aggregate bildet.

8. Verfahren nach einem der vorherigen Ansprüche,
**dadurch gekennzeichnet, dass** die nachzuweisende Substanz ein Prion-Protein oder ein Mistel-Lektin ist.

9. Verfahren nach einem der Ansprüche 7-8,
**dadurch gekennzeichnet, dass** der Schritt d) bzw. die Schritte f) und/oder g) im wesentlichen frei von Detergenzien durchgeführt wird bzw. werden.

10. Testassay um in einer wässrigen Lösung eine oder mehrere Substanzen nachzuweisen,
**dadurch gekennzeichnet, dass** mit der wässrigen Lösung mehrere Verfahren nach einem der vorherigen Ansprüche gleichzeitig oder nacheinander durchgeführt werden, wobei sich die Verfahren jeweils voneinander unterscheiden in
a) den ersten und jeweils zugeordneten zweiten Nucleinsäuren und/oder
b) den dritten Nucleinsäuren.

11. Verwendung eines Fänger-Reagens, umfassend eine zweite Nucleinsäure und ein mit dieser verbundenes erstes Bindungsmolekül, das an eine nachzuweisende Substanz binden kann, zur Durchführung eines Verfahrens gemäß einem der vorherigen Ansprüche.

12. Nachweiskonjugat, erhältlich durch ein Verfahren umfassend die Schritte b), c) und d) nach einem der Ansprüche 1-10.

13. Kit zum Herstellen eines Nachweiskonjugats nach Anspruch 12, umfassend
a) ein erstes Bindungsmolekül und gegebenenfalls ein zweites Bindungsmolekül,
b) eine zweite und eine dritte Nucleinsäure,
c) Mittel zum Verbinden des ersten Bindungsmoleküls mit jeweils der zweiten bzw. dritten Nucleinsäure, bzw. Mittel zum Verbinden des ersten Bindungsmoleküls mit der zweiten Nucleinsäure und Mittel zum Verbinden des zweiten Bindungsmoleküls mit der dritten Nucleinsäure.
